(19) Europäisches Patentamt
European Patent Office
Office européen des brevets

(11) Veröffentlichungsnummer : **0 021 206**
**B1**

(12) **EUROPÄISCHE PATENTSCHRIFT**

(45) Veröffentlichungstag der Patentschrift : **22.06.83**

(51) Int. Cl.³ : **C 07 D457/12**, A 61 K 31/40

(21) Anmeldenummer : **80103177.4**

(22) Anmeldetag : **07.06.80**

# (54) Neue (Ergolin-yl)-N', N'-diäthylharnstoff-Derivate, ihre Herstellung und sie enthaltende Arzneimittel.

(30) Priorität : **13.06.79 DE 2924102**
**28.04.80 DE 3016691**

(43) Veröffentlichungstag der Anmeldung : **07.01.81 Patentblatt 81/01**

(45) Bekanntmachung des Hinweises auf die Patenterteilung : **22.06.83 Patentblatt 83/25**

(84) Benannte Vertragsstaaten : **AT BE CH DE FR GB IT LI LU NL SE**

(56) Entgegenhaltungen :
**AT B 2 774 477**
**DE A 2 238 540**

**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Horowski, Reinhard, Dr.**
**Am Rosenanger 32**
**D-1000 Berlin 28 (DE)**
Erfinder : **Kehr, Wolfgang, Dr.**
**Biedermann Weg 11**
**D-1000 Berlin 19 (DE)**
Erfinder : **Sauer, Gerhard, Dr.**
**Königsbacher Zeile 41A**
**D-1000 Berlin 28 (DE)**
Erfinder : **Eder, Ulrich, Dr.**
**Zeltinger Strasse 17**
**D-1000 Berlin 28 (DE)**
Erfinder : **Lorenz, Hans Peter, Dr.**
**Residenzstrasse 17/18**
**D-1000 Berlin 51 (DE)**

**EP 0 021 206 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Neue (Ergolin-yl)-N', N'-diäthylharnstoff-Derivate, ihre Herstellung und sie enthaltende Arzneimittel

Die Erfindung betrifft neue (Ergolin-yl)-N'.N'-diäthylharnstoffderivate gemäß Anspruch 2, deren Herstellung nach an sich bekannten Methoden und Arzneimittel auf Basis dieser Verbindungen.

Die Alkylreste mit bis zu 6 C-Atomen sind solche, die sich von den aliphatischen Kohlenwasserstoffen ableiten, wie z. B. Äthyl, n-Propyl, Isopropyl, n-Butyl, Isobutyl, tert.-Butyl, n-Pentyl usw. Alkylreste, soweit sie am $N^6$-Atom stehen, können aber auch durch Säurefunktionen, wie R'-Oxycarbonyl oder Carbonitril, wobei R' niederes Alkyl bedeutet, substituiert sein.

Die Salze der erfindungsgemäßen Verbindungen sind Säureadditionssalze und leiten sich von physiologisch unbedenklichen Säuren ab. Solche physiologisch unbedenklichen Säuren sind anorganische Säuren, wie beispielsweise Chlorwasserstoffsäure, Salpetersäure, Phosphorsäure, Schwefelsäure, Bromwasserstoffsäure, Jodwasserstoffsäure, salpetrige Säure oder phosphorige Säure, oder organische Säuren, wie beispielsweise aliphatische Mono- oder Dicarbonsäuren, phenylsubstituierte Alkancarbonsäuren, Hydroxyalkancarbonsäuren oder Alkandicarbonsäuren, aromatische Säuren oder aliphatische oder aromatische Sulfonsäuren, Physiologisch unbedenkliche Salze dieser Säuren sind daher z. B. das Sulfat, Pyrosulfat, Bisulfat, Sulfit, Bisulfit, Nitrat, Phosphat, Monohydrogenphosphat, Dihydrogenphosphat, Metaphosphat, Pyrophosphat, Chlorid, Bromid, Jodid, Fluorid, Acetat, Propionat, Decanoat, Caprylat, Acrylat, Formiat, Isobutyrat, Caproat, Heptanoat, Propiolat, Malonat, Succinat, Suberat, Sebacat, Fumarat, Maleat, Mandelat, Butin-1.4-dioat, Hexin-1.6-dioat, Benzoat, Chlorbenzoat, Methylbenzoat, Dinitrobenzoat, Hydroxybenzoat, Methoxybenzoat, Phthalat, Terephthalat, Benzolsulfonat, Toluolsulfonat, Chlorbenzolsulfonat, Xylolsulfonat, Phenylacetat, Phenylpropionat, Phenylbutyrat, Citrat, Lactat, β-Hydroxybutyrat, Glycollat, Malat, Tartrat, Methansulfonat, Propansulfonat, Naphthalin-1-sulfonat oder Naphthalin-2-sulfonat.

Die erfindungsgemäßen Verbindungen besitzen eine ausgeprägte dopaminerge Wirksamkeit und sind überraschenderweise dem bekannten Lisurid-hydrogenmaleat (AT-B-2 774 477) bzw. dessen in 9.10-Stellung hydrierter Form N-(D-6-Methyl-8-isoergolin-I-yl)-N'.N'-diethylharnstoff (DE-A-2 238 548) in ihrer Wirkung überlegen.

Die Wirksamkeit der erfindungsgemäßen Substanzen wurde durch Bestimmung der Prolaktinkonzentration im Serum von Kleinnagern nach i.p.-Applikation radioimmunologisch bestimmt und das Verhalten der Versuchstiere analysiert. Nach den Untersuchungen von Andén et al. kann das Auftreten von stereotypen Bewegungsabläufen bei Maus und Ratte wie Kauen, Nagen und Lecken, auch nach Depletion der Monoaminspeicher mit Reserpin (5 mg/kg i.p. 24 Stunden vor Prüfung), zusammen mit der Aufhebung der durch Reserpin hervorgerufenen Immobilität direkt als Zeichen der Dopaminrezeptor-stimulierenden Wirkung gewertet werden (Andén, N.-E., Strömbom, U. und Svensson, T. H.: Dopamine ans noradrenaline receptor stimulation : reversal of reserpine-induced suppression of motor activity, Psychopharmacologia *29* : 289, 1973).

Die erfindungsgemäßen Verbindungen eignen sich somit beispielsweise zur Laktationshemmung und zur Behandlung des Parkinsonismus.

Zur Verwendung der erfindungsgemäßen Verbindungen als Arzneimittel werden diese in die Form eines pharmazeutischen Präparats gebracht, das neben dem Wirkstoff ein für die enterale oder parenterale Applikation geeignete pharmazeutische, organische oder anorganische inerte Trägermaterialien, wie z. B. Wasser, Gelatine, Gummi arabicum,Milchzucker, Stärke, Magnesiumstearat, Talk, pflanzliche Öle, Polyalkylenglykole usw. enthält. Die pharmazeutischen Präparat können in fester Form, z. B. als Tabletten, Dragées, Suppositorien, Kapseln oder in flüssiger Form, z. B. als Lösungen, Suspensionen oder Emulsionen vorliegen, Gegebenenfalls enthalten sie darüber hinaus Hilfsstoffe wie Konservierungs-, Stabilisierungs-, Netzmittel oder Emulgatoren, Salze zur Veränderung des osmotischen Druckes oder Puffer.

Die Erfindung betrifft weiterhin ein Verfahren zur Herstellung der erfindungsgemäßen Verbindungen, welches dadurch gekennzeichnet ist, daß man in an sich bekannter Weise $N^6$-Alkylierte Lysergsäuremethylester mit Hydrazin zum Hydrazid umsetzt, mit salpetriger Säure in das Azid überführt, durch Erhitzen das Isocyanat bildet, anschließend mit Diäthylamin zur Reaktion bringt und gegebenenfalls die 9.10-Doppelbindung hydriert und gewünschtenfalls die so erhaltenen Verbindungen in ihre Salze überführt.

Zur Durchführung des erfindungsgemäßen Verfahrens werden in der ersten Stufe die N-alkylierten Lysergsäure-methylester mit wasserfreiem Hydrazin zu den entsprechenden Hydraziden umgesetzt, wobei jedoch eine Auftrennung der Isomeren unterbleibt.

In der zweiten Stufe wird das so erhaltene Hydrazid mit salpetriger Säure in das Säureazid überführt und das wässrige Reaktionsgemisch mit einem Puffer wie Natriumhydrocarbonat, Dinatriumhydrogenphosphat, Natriumacetat, Kaliumborat oder Ammoniak versetzt und mit Toluol extrahiert.

In der dritten Stufe wird die Toluolphase auf Temperaturen oberhalb Raumtemperatur, vorzugsweise 70° bis zur Siedetemperatur des Reaktionsgemisches, erwärmt, wobei sich das entsprechende Isocyanat bildet.

In der vierten Reaktionsstufe wird das so gebildete Isocyanat mit Diäthylamin bei Raumtemperatur zur Reaktion gebracht, wobei ein isomeres Gemisch von N'.N'-Diäthylharnstoffderivaten entsteht, die zweck-mäßigerweise chromatographisch getrennt werden.

Will man gegebenenfalls zu in 9.10-Stellung gesättigten Verbindungen gelangen, werden die zuvor erhaltenen Verfahrensprodukte in an sich bekannter Weise hydriert. Geeignete Methoden sind Hydrierungen mit Wasserstoff in Gegenwart von Palladium auf Kohle oder anderen geeigneten Trägern wie Kalk, in Gegenwart von Platin z. B. in Form von Platinmohr oder in Gegenwart von Nickel wie z. B. in Form von Raney-Nickel. Anschließend wird chromatographisch gereinigt bzw. in die Isomeren aufgetrennt.

Die so erhaltenen Verbindungen werden entweder als freie Basen oder in Form ihrer Säureadditionssalze, die gewünschtenfalls durch Umsetzung mit einer physiologisch verträglichen Säure, wie z. B. Weinsäure, Maleinsäure oder Benzoesäure, erhalten werden, durch Umkristallisation und/oder Chromatographie gereinigt.

Zur Bildung von Salzen wird die erhaltene Verbindung in wenig Methanol gelöst und mit einer konzentrierten Lösung der gewünschten organischen Säure in Methanol bei Raumtemperatur versetzt.

Die Ausgangsstoffe für das erfindungsgemäße Verfahren können, soweit sie nicht bekannt sind, in Analogie zu bekannten Methoden hergestellt werden (T. Fehr et al., Helv. Chim. Acta *53* (1970) 2197 bzw. J. Krepelka et al., Coll. Czech. Chem. Commun. *42* (1977) 1209).

Die nicht bekannten Ausgangsstoffe können z. B. nach folgender allgemeiner Arbeitsvorschrift hergestellt werden :

Man löst 1 mMol 6-Nor-lysergsäuremethylester in 10 ml Dimethylformamid, Nitromethan oder Acetonitril, gibt 420 mg wasserfreies Kaliumcarbonat (3 mMol) und 1,6 mMol des Alkylierungsmittels, z. B. als Halogenid R‴Hal, zu und erwärmt 1 bis 8 Stunden lang auf Temperaturen bis zu 50 °C. Dann destilliert man das Lösungsmittel im Vakuum weitgehend ab, verteilt den Rückstand zwischen Chloroform und Wasser und schüttelt die Wasserphase mehrmals mit Chloroform aus. Die organischen Phasen werden mit Wasser gewaschen, mit Magnesiumsulfat getrocknet und eingedampft. Das Rohprodukt stellt meist ein öliges Gemisch der 8 Isomeren dar, das für die folgende Reaktion genügend rein ist. Durch Filtration über Kieselgel werden dunkle Anteile entfernt.

Die nachfolgenden Beispiele sollen das erfindungsgemäße Verfahren erläutern.

Die Ir-Spektren wurden in KBr, die UV-Spektren in Methanol als Lösungsmittel und die NMR-Spektren, soweit nicht anders angegeben, in $CDCl_3$ gemessen.

## Beispiel 1

Man löst 2,9 g 6-Nor-6-äthyl-(iso)-lysergsäuremethylester in 100 ml wasserfreiem Hydrazin und erwärmt eine Stunde auf 50 °C. Dann kühlt man ab, verdünnt mit 300 ml Chloroform und schüttelt mit einer gesättigten Kochsalzlösung aus. Die organische Phase wird getrocknet und eingedampft. Man erhält 3,1 g isomeres 6-Nor-6-äthyl-(iso)-lysergsäure-hydrazid, das ohne Aufarbeitung in 50 ml 0,2 n Salzsäure gelöst, unter Eiskühlung mit 10 ml 1 n Natriumnitritlösung und 55 ml 0,2 n Salzsäure versetzt wird. Nach etwa 5 Minuten verteilt man die Mischung zwischen Toluol und Natriumbicarbonatlösung, schüttelt die wässrige Phase mit weiterem Toluol aus und trocknet mit Natrium-sulfat. Dann wird die Toluolphase 15 Minuten auf 80 °C erwärmt und mit 10 ml destilliertem Diethylamin bei Raumtemperatur eine Stunde gerührt. Nach Eindampfen verbleiben 3,8 g des isomeren 3-(9.10-Didehydro-6-äthyl-8-ergolinyl)-1.1-diäthyl-harnstoffs.

Zur Trennung des Isomerengemisches wird an Kieselgel mit einem Gradienten von Methanol und Chloroform chromatographiert und man erhält 1,2 g schneller laufende Komponente, die die 8α-konfigurierte Verbindung 3-(9.10-Didehydro-6-ethyl-8α-ergolinyl)-1.1-dimethyl-harnstoff

IR : 3 250, 1 638, 1 505 $cm^{-1}$ ist.

UV : $\lambda_{max}$ = 219 (15 700), 225 (15 700), 241 (14 800), 310 (6 150)

NMR : δ = 1,11 (t, J = Hz, 9H), 6,51 (m, 1H), 6,88 (m, 1H), 8,52 (s, 1H).

Dieses wird in wenig Methanol gelöst und mit einer konzentrierten Lösung von 0,6 g Maleinsäure in Methanol bei Raumtemperatur versetzt. Man isoliert 1,4 g kristallines 3-(9.10-Didehydro-6-äthyl-8α-ergolinyl)-1.1-diäthylharnstoff-hydrogenmaleat.

Die bei der Chromatographie langsamer laufende Komponente (1,1 g Rohprodukt als 8β-konfigurierte Verbindung 3-(9.10-Didehydro-6-ethyl-8β-ergolinyl)-1.1-diethylharnstoff

IR : 3 240, 1 625, 1 510 $cm^{-1}$

UV : $\lambda_{max}$ = 241 (14 200), 310 (6 150)

NMR : δ = 6,30 (m, 1H), 6,87 (m, 1H), 8,36 (s, 1H) wird ebenfalls in wenig Methanol gelöst, mit einer konzentrierten Lösung von 0,6 g Maleinsäure versetzt und kristallisiert. Man erhält 1,0 g 3-(9.10-Didehydro-6-äthyl-8β-ergolinyl)-1.1-diäthylharnstoff-hydrogenmaleat.

## Beispiel 2

Aus 3,1 g 6-Nor-6-n-propyl-(iso)-lysergsäuremethylester erhält man bei einstündigem Stehen bei Raumtemperatur in 100 ml wasserfreiem Hydrazinhydrat nach einer Stunde und einer Aufarbeitung wie im Beispiel 1 beschrieben 3,2 g isomeres 6-Nor-6-n-propyl-(iso)-lysergsäure-hydrazid, das wie im Versuch 1 beschrieben, umgesetzt und aufgearbeitet wird, wobei man 3,2 g des isomeren 3-(9.10-Didehydro-6-n-propyl-8-ergolinyl)-1.1-diäthylharnstoffs erhält.

Dieses Isomerengemisch wird and Kieselgel mit Methanol und Chloroform chromatographiert, wobei

wieder die schneller laufenden Anteile die 8α-Verbindung 3-(9.10-Didehydro-6-n-propyl-8α-ergolinyl)-1.1-diethylharnstoff

IR : 3 420, 1 630, 1 505 $cm^{-1}$

UV : $\lambda_{max}$ = 216 (17 900), 240 (16 900), 310 (7 140)

NMR : δ = 6,54 (m, 1H), 6,88 (m, 1H), 7,97 (s, 1H)

enthalten. Diese wird in wenig Methanol gelöst, mit einer Lösung von 0,5 g L-Weinsäure versetzt und bei 0 °C kristallisiert. Man erhält 1,2 g 3-(9.10-Didehydro-6-n-propyl-8α-ergolinyl)-1.1-diäthylharnstofftartrat.

Der langsamer laufende Anteil, der 3-(9.10)-Didehydro-6-n-propyl-8β-ergolinyl)-1.1-diäthylharnstoff darstellt, wird ebenfalls mit 0,5 g L-Weinsäure in methanolischer Lösung in das 3-(9.10-Didehydro-6-n-propyl-8β-ergolinyl)-1.1-diäthylharnstofftartrat überführt.

IR : 1 614, 1 525 $cm^{-1}$

NMR : (d-MeOH) δ = 4,02 (m, 1H), 4,43 (m, 1H), 6,40 (m, 1H) 7,03 (m, 1H).

## Beispiel 3

In gleicher Weise, wie im Beispiel 1 beschrieben, erhält man aus 310 mg 6-Nor-6-isopropyl-(iso)-lysergsäuremethyl-ester das gewünschte isomere 6-Nor-6-iso-propyl-(iso)-lysergsäurehydrazid in 290 mg Ausbeute, das wie im Beispiel 1 beschrieben umgesetzt und aufgearbeitet wird, wobei man 1/10 der in Beispiel 1 angegebenen Reagentien verwendet. Das Rohprodukt von 335 mg 3-(9.10-Didehydro-6-isopropyl-8-ergolinyl)-1.1-diäthylharnstoff wird durch präparative Schichtchromatographie aufgetrennt. Der schneller laufende Anteil (98 mg) wird mit 50 mg Maleinsäure als 3-(9.10-Didehydro-6-isopropyl-8α-ergolinyl)-1.1-diäthylharnstoff-hydrogenmaleat kristallisiert.

Die langsamer laufende Substanz, die 3-(9.10-Didehydro-6-isopropyl-8β-ergolinyl)-1.1-diäthylharnstoff darstellt, wird mit Methansulfonsäure als 3-(9.10-Didehydro-6-isopropyl-8β-ergolinyl)-1.1-diäthylharnstoff-methansulfonat kristallisiert.

## Beispiel 4

Man setzt 324 mg 6-Nor-6-n-butyl-(iso)-lysergsäuremethyl-ester, wie im Beispiel 3 beschrieben, zum Isomerengemisch der Hydrazide um und erhält eine Rohausbeute von 330 mg 6-Nor-6-n-butyl-(iso)-lysergsäurehydrazid, das — wie im Beispiel 3 beschrieben, in das Gemisch der 3-(9.10-Didehydro-6-n-butyl-8-ergolinyl)-1.1-diäthylharnstoffe überführt und chromatographisch getrennt wird. Mit 50 mg Maleinsäure erhält man aus der schneller laufenden Komponente die 3-(9.10-Didehydro-6-n-butyl-8α-ergolinyl)-1.1-diethylharnstoff

IR : 3 250, 1 650, 1 505 $cm^{-1}$

UV : $\lambda_{max}$ = 241 (17 100), 310 (7 130)

NMR : δ = 1,08 (t, J = 7Hz, 6H), 1,17 (t, J = 7Hz, 3H), 6,58 (m, 1H), 6,93 (m, 1H), 8,28 (s, 1H) darstellt, 103 mg 3-(9.10-Didehydro-6-n-butyl-8α-ergolinyl)-1.1-diäthylharnstoff-hydrogenmaleat.

Die langsamer laufende Komponente der Chromatographie, die 3-(9.10-Didehydro-6-n-butyl-8β-ergolinyl)-1.1-diäthylharnstoff

IR : 3 270, 1 660, 1 505 $cm^{-1}$

UV : $\lambda_{max}$ = 219 (21 300), 225 (21 200), 241 (19 500), 310 (8 390)

NMR : δ = 6,36 (m, 1H), 6,93 (m, 1H), 8,20 (s, 1H) darstellt, liefert mit Weinsäure in Methanol 120 mg 3-(9.10-Didehydro-6-n-butyl-8β-ergolinyl)-1.1-diäthylharnstoff-tartrat.

## Beispiel 5

Man löst 1,0 g 3-(9.10-Didehydro-6-äthyl-8β-ergolinyl)- 1.1-diäthylharnstoff in 20 ml Methanol, gibt 100 mg Palladium/Kohle zu und hydriert bei Raumtemperatur und Normaldruck, bis die berechnete Menge Wasserstoff aufgenommen ist. Man filtriert den Katalysator ab, engt ein und gibt bis zur deutlich sauren Reaktion 2 n Phosphorsäure zu. Nach Umkristallisation aus Methanol erhält man 0,9 g 3-(6-Äthyl-8β-ergolin-l-yl)-1.1-diäthylharnstoffphosphat.

IR : 1 620 $cm^{-1}$

UV : $\lambda_{max}$ = 225 (27 000), 285 (6 200)

## Beispiel 6

1,0 g 3-(9.10-Didehydro-6-äthyl-8α-ergolinyl)-1.1-diäthylharnstoff werden in 30 ml Methanol gelöst, mit etwa 1 g Raney-Nickel versetzt und bei Raumtemperatur und einem Wasserstoffdruck von 35 atü hydriert. Man filtriert vom Katalysator ab, engt ein und erhält nach Chromatographie des Rohproduktes an Kieselgel mit einem Gradienten von Chloroform und Methanol 0,5 g 3-(6-Äthyl-8α-ergolin-l-yl)-1.1-diäthylharnstoff, der mit Phosphorsäure in das kristalline Salz überführt wird. Nach Umkristallisation aus Methanol erhält man 0,4 g 3-(6-Äthyl-8α-ergolin-l-yl)-1.1-diäthylharnstoff-phosphat.

IR : 1 620 $cm^{-1}$

UV : $\lambda_{max}$ = 223 (28 000), 281 (6 100), 292 (5 450)

## Beispiel 7

Wie im Beispiel 5 beschrieben, wird 1,0 g 3-(9.10-Didehydro-6-n-propyl-8β-ergolinyl)-1.1-diäthylharnstoff in Dioxan hydriert und aufgearbeitet, wobei man nach Umsetzung mit Weinsäure 0,8 g 3-(6-n-Propyl-8β-ergolin-I-yl)-1.1-diäthylharnstofftartrat isoliert.

IR : 1 620 $cm^{-1}$

UV : $\lambda_{max}$ = 225 (27 000), 285 (6 200)

## Beispiel 8

Wie im Beispiel 6 beschrieben, werden 2,0 g 3-(9.10-Didehydro-6-n-propyl-8α-ergolinyl)-1.1-diäthylharnstoff hydriert und aufgearbeitet und als Salz der Phosphorsäure isoliert. Man erhält 0,8 g 3-(6-n-Propyl-8α-ergolin-I-yl)-1.1-diäthylharnstoff-phosphat. Mit Weinsäure erhält man das entsprechende Tartrat 3-(6-n-Propyl-8α-ergolin-I-yl)-1.1-diethylharnstoff-tartrat

IR : 1 620 $cm^{-1}$

UV : $\lambda_{max}$ = 223 (28 900), 281 (6 400), 292 (5 390)

NMR : (d-Py) : δ = 0,88 (t, J = 7Hz, 3H), 1,12 (t, J = 7 Hz, 6H), 5,41 (m, 1H), 11,55 (s, 1H).

## Beispiel 9

Man hydriert, wie im Beispiel 5 beschrieben, 1,0 g 3-(9.10-Didehydro-6-isopropyl-8β-ergolinyl)-1.1-diäthylharnstoff und erhält mit Maleinsäure 3-(6-Isopropyl-8β-ergolin-I-yl)-1.1-diäthylharnstoff-hydrogenmaleat in einer Ausbeute von 0,8 g.

## Beispiel 10

Die Hydrierung von 1,5 g 3-(9.10-Didehydro-6-isopropyl-8α-ergolinyl)-1.1-diäthylharnstoff, wie im Beispiel 6 beschrieben, liefert nach Chromatographie 3-(6-Isopropyl-8α-ergolin-I-yl)-1.1-diäthylharnstoff und man erhält nach Kristallisation mit Phosphorsäure 0,5 g 3-(6-Isopropyl-8α-ergolin-I-yl)-1.1-diäthylharnstoff-phosphat.

## Beispiel 11

0,5 g 3-(9.10-Didehydro-6-n-butyl-8β-ergolinyl)-1.1-diäthylharnstoff werden, wie in Beispiel 5 beschrieben, hydriert und kristallisiert. Ausbeute : 0,5 g 3-(6-n-Butyl-8β-ergolin-I-yl)-1.1-diäthylharnstoff-phosphat.

IR : 1 625 $cm^{-1}$

UV : $\lambda_{max}$ = 225 (26 500), 286 (6 100)

## Beispiel 12

Wie im Beispiel 6 beschrieben, werden 1,5 g 3-(9.10-Didehydro-6-n-butyl-8α-ergolinyl)-1.1-diäthylharnstoff hydriert und kristallisiert, wobei 0,6 g 3-(6-n-Butyl-8α-ergolin-I-yl)-1.1-diäthylharnstoff-phosphat erhalten werden.

IR : 1 620 $cm^{-1}$

UV : $\lambda_{max}$ = 223 (28 000), 280 (6 200), 292 (5 400)

## Beispiel 13

Die Verbindungen werden nach der folgenden allgemeinen Vorschrift hergestellt :

Man löst 10 mMol des als Ausgangsmaterial verwendeten $N^6$-Alkyl-lysergsäuremethylesters (Isomerengemisch) in 100 ml wasserfreiem Hydrazin und erwärmt 16 Stunden auf 50 °C. Dann kühlt man ab, verdünnt mit 300 ml Chloroform und schüttelt mit einer gesättigten Kochsalzlösung aus. Die organische Phase wird getrocknet und eingedampft. Das so erhaltene Hydrazid ist ein Isomerengemisch und wird ohne Reinigung in die nächste Stufe eingesetzt.

Man löst 1 g Hydrazid in 10 ml Tetrahydrofuran, versetzt unter Eiskühlung mit 12 ml In Salzsäure und gibt nach 10 min Rühren 3,6 ml In Natriumnitritlösung und 7,2 ml In Salzsäure zu und rührt weitere 10 min im Eisbad. Man überschichtet mit 100 ml Toluol, tropft unter starkem Rühren 50 ml gesättigte Natriumbicarbonatlösung zu und trennt die organische Phase ab. Die Wasserphase wird zweimal mit 50 ml Toluol ausgeschüttelt, alle organischen Phasen getrocknet, vereinigt und unter Argon 15 min in einem Bad von 100 °C erhitzt. Danach kühlt man die Lösung auf Raumtemperatur ab, versetzt mit 3 ml Diethylamin und rührt 1 Stunde bei Raumtemperatur. Nach Eindampfen werden die isomeren 3-(9.10-Didehydro-6-alkyl-8-ergolinyl)-1.1-diethyl-harnstoffe erhalten. Zur Trennung des Isomerengemisches wird an Kieselgel mit einem Gradienten von Methanol und Tetrachlormethan chromatographiert, die schneller

laufende Komponente ist die 8α- und die langsamer laufende die 8β-Verbindung.
Im einzelnen werden hergestellt :

3-(9.10-Didehydro-6-(2-propen-1-yl)-8α-ergolinyl)-1.1-diethylharnstoff, Ausbeute : 52 % d. Th.
IR : 3 300, 1 630, 1 505 $cm^{-1}$
UV : $\lambda_{max}$ = 216 (17 000), 241 (15 100), 310 (6 900)
NMR : $\delta$ = (6,55 (m, 1H), 6,90 (m, 1H), 8,20 (s, 1H) und
3-(9.10-Didehydro-6-(2-propen-1-yl)-8β-ergolinyl)-1.1-diethylharnstoff, Ausbeute : 15 % d. Th.
IR : 3 300, 1 625, 1 505 $cm^{-1}$
UV : $\lambda_{max}$ = 241 (16 500), 310 (6 500)
NMR : $\delta$ = 6,43 (m, 1H), 6,92 (m, 1H), 805 (s, 1H).
aus
9,10-Didehydro-6-(2-propen-1-yl)-ergolin-8-carbonsäuremethylester.
IR : 3 405 ; 1 730 $cm^{-1}$
NMR : $\delta$ = 3,78 (s, 3H) bzw. 3,85 (s, 3H), 6,61 (m, 1H), 6,92 (m, 1H), 8,07 (s, 1H).
3-(9.10-Didehydro-6-(2-propin-1-yl)-8α-ergolinyl)-1.1-diethylharnstoff, Ausbeute : 48 %
IR : 3 250, 1 638, 1 505 $cm^{-1}$
UV : $\lambda_{max}$ = 240 (14 000), 310 (5 900)
NMR : $\delta$ = 6,50 (m, 1H), 6,90 (m, 1H), 8,10 (s, 1H) und 3-(9.10-Didehydro-6-(2-propin-1-yl)-8β-ergolinyl)-1.1-diethylharnstoff, Ausbeute : 15 %
IR : 3 250, 1 650, 1 510 $cm^{-1}$
UV : $\lambda_{max}$ = 219 (21 000), 225 (20 800), 241 (18 800), 310 (8 100)
aus
9.10-Didehydro-6-(2-propin-1-yl)-ergolin-8-carbonsäure-methylester
IR : 3 410, 1 725 $cm^{-1}$
NMR : $\delta$ = 3,73 (s, 3H), bzw. 3,81 (s, 3H), 6,57 (m, 1H), 6,90 (m, 1H), 7,98 (m, 1H) ;
3-(9.10-Didehydro-8α-(3.3-diethylureido)-6-ergolin)-propionsäureethylester, Ausbeute : 36 %
IR : 3 290, 1 655, 1 505 $cm^{-1}$
UV : $\lambda_{max}$ = 240 (18 000), 310 (7 500) und
3-(9.10-Didehydro-8β-(3.3-diethylureido)-6-ergolin)-propionsäureethylester, Ausbeute : 15 %
IR : 3 270, 1 650, 1 510 $cm^{-1}$
UV : $\lambda_{max}$ = 241 (16 900), 310 (6 000)
aus
3-(9.10-Didehydro-8-methoxycarbonyl-6-ergolin)-propionsäureethylester
IR : 3 405, 1 730 $cm^{-1}$
UV : $\lambda_{max}$ = 222 (20 600), 309 (6 700)
NMR : $\delta$ = 1,33 (t, J = 7Hz, 3H), 3,75 (s, 3H), bzw. 3,82 (s, 3H), 6,57 (m, 1H), 6,92 (m, 1H), 7,97 (s, 1H) ;
3-(8α-(3.3-Diethylureido)-6-ergolin)-propionsäureethylester, Ausbeute : 45 %
IR : 1 625 $cm^{-1}$
UV : $\lambda_{max}$ = 225 (26 500) 281 (5 500), 290 (5 200)
NMR : $\delta$ = 6,92 (m, 1H), 8,30 (s, 1H) ;
und
3-(8β-(3.3-Diethylureido)-6-ergolin)-propionsäureethylester, Ausbeute : 72 %
IR : 3 300, 1 630 $cm^{-1}$
UV : $\lambda_{max}$ = 224 (25 000), 285 (5 800) ;
3-(9.10-Didehydro-8α-(3.3-diethylureido)-6-ergolin)-propionitril, Ausbeute : 43 %
IR : 3 420, 2 245, 1 630, 1 505 $cm^{-1}$
UV : $\lambda_{max}$ = 220 (21 000), 307 (7 100)
NMR : $\delta$ = 6,54 (m, 1H), 6,95 (m, 1H), 8,10 (s, 1H)
und
3-(9.10-Didehydro-8β-(3.3-diethylureido)-6-ergolin)-propionitril, Ausbeute : 19 %
IR : 3 400, 2 250, 1 640, 1 505 $cm^{-1}$
UV : $\lambda_{max}$ = 223 (20 100), 310 (7 000)
aus
6-(2-Cyanethyl)-9.10-didehydro-ergolin-8-carbonsäuremethylester
IR : 3 410, 2 250, 1 730 $cm^{-1}$
UV : $\lambda_{max}$ = 223 (22 000), 307 (7 350)
NMR : $\delta$ = 3,72 (s, 3H) bzw. 3,80 (s, 3H), 6,51 (m, 1H), 6,87 (m, 1H), 7,95 (s. 1H) ;
3-(6-Cyclopropyl-9.10-didehydro-8α-ergolinyl)-1.1-diethylharnstoff, Ausbeute : 35 %
IR : 3 250, 1 635, 1 505 $cm^{-1}$
UV : $\lambda_{max}$ = 218 (18 300), 241 (16 900), 310 (6 500)
und
3-(6-Cyclopropyl-9.10-didehydro-8β-ergolinyl)-1.1-diethylharnstoff, Ausbeute : 18 %
IR : 3 250, 1 630, 1 505 $cm^{-1}$
UV : $\lambda_{max}$ = 242 (17 000), 310 (6 300)

aus

6-Cyclopropyl-9.10-didehydro-ergolin-8-carbonsäuremethyl-ester
IR : 3 400, 1 735 $cm^{-1}$
UV : $\lambda_{max}$ = 225 (20 500), 230 (19 100), 310 (7 900)
NMR : $\delta$ = 3,79 (s, 3H), 6,55 (m, 1H), 6,90 (m, 1H), 8,10 (s. 1H) ;
3-(6-Cyclopropyl)-8α-ergolinyl)-1.1-diethylharnstoff Ausbeute : 40 %
IR : 3 350, 1 620 $cm^{-1}$
UV : $\lambda_{max}$ = 224 (26 000), 281 (6 200), 292 (5 200)

und

3-(6-Cyclopropyl-8β-ergolinyl)-1.1-diethylharnstoff Ausbeute : 68 %
IR : 3 300, 1 615 $cm^{-1}$
UV : $\lambda_{max}$ = 225 (27 000), 283 (5 800), 295 (5 100) ;
3-(6-Cyclobutyl-9.10-didehydro-8α-ergolinyl)-1.1-diethylharnstoff, Ausbeute : 39 %
IR : 3 350, 1 640, 1 505 $cm^{-1}$
UV : $\lambda_{max}$ = 219 (19 400), 241 (17 000), 310 (7 050)

und

3-(6-Cyclobutyl-9.10-didehydro-8β-ergolinyl)-1.1-diethylharnstoff, Ausbeute : 13 %
IR : 3 250, 1 640, 1 510 $cm^{-1}$
UV : $\lambda_{max}$ = 241 (14 300), 310 (6 150)

aus

6-Cyclobutyl-9.10-didehydro-ergolin-8-carbonsäuremethylester
IR : 3 300, 1 740
UV : $\lambda_{max}$ = 227 (21 000), 240 (17 300), 310 (6 900)
NMR : $\delta$ = 3,70 (s, 3H), bzw. 3,78 (s, 3H), 6,57 (m, 1H), 6,87 (m, 1H), 8,05 (s, 1H) ;
3-((6-Cyclopropyl-methyl)-9.10-didehydro-8α-ergolinyl)-1.1-diethylharnstoff, Ausbeute : 40 %
IR : 3 220, 1 630, 1 505 $cm^{-1}$
UV : $\lambda_{max}$ = 216 (17 800), 240 (16 900), 310 (7 040) und
3-((6-Cyclopropyl)-methyl)-9.10-didehydro-8β-ergolinyl)-1.1-diethylharnstoff
IR : 3 280, 1 630, 1 505 $cm^{-1}$
UV : $\lambda_{max}$ = 219 (21 000), 225 (21 100), 241 (19 500), 310 (8 100)

aus

6-(Cyclopropyl-methyl)-9.10-didehydro-ergolin-8-carbonsäure-methylester
IR : 3 270, 1 740 $cm^{-1}$
UV : $\lambda_{max}$ = 226 (19 500), 240 (17 100), 310 (7 050).

**Ansprüche**

1. Verfahren zur Herstellung von (Ergolin-yl)-N'.N'-diäthylharnstoffderivaten der allgemeinen Formel

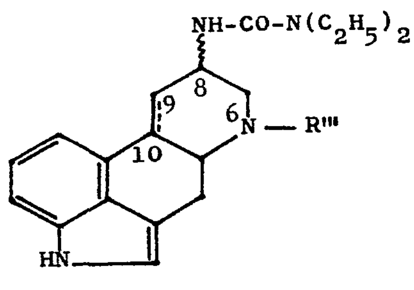

und deren Salzen, worin

R''' einen Alkylrest mit 2-6 C-Atomen und

$-(CH_2)_n-CH=CH_2$, $-(CH_2)_n-C\equiv CH$, $-(CH_2)_n-COOR'$,

$-(CH_2)_n-CN$ und 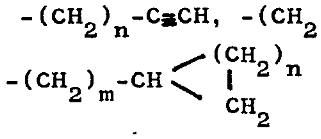 ,

wobei

n = 1 und 2,
m = 0 und 1 und
R' = niederes Alkyl mit bis zu 6 C-Atomen bedeuten,

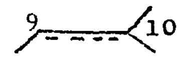

eine CC-Einfach- oder CC-Doppelbindung bedeuten und der 8-ständige Harnstoffrest α- oder β-ständig sein kann, dadurch gekennzeichnet, daß man in an sich bekannter Weise $N^6$-alkylierte Lysergsäuremethylester mit Hydrazin zum Hydrazid umsetzt, mit salpetriger Säure in das Azid überführt, durch Erhitzen das Isocyanat bildet, anschließend mit Diäthylamin zur Reaktion bringt und gegebenenfalls die 9.10-Doppelbindung hydriert und gewünschtenfalls die so erhaltenen Verbindungen in ihre Salze überführt.

2. (Ergolin-yl)-N'. N'-diäthylharnstoffderivate der allgemeinen Formel

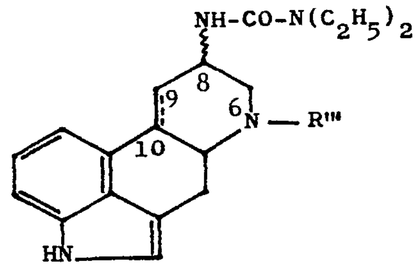

und deren salze, worin

R''' einen Alkylrest mit 2-6 C-Atomen und

$-(CH_2)_n-CH=CH_2$, $-(CH_2)_n-C \equiv CH$, $-(CH_2)_n-COOR'$,

$-(CH_2)_n-CN$ und 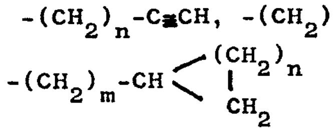 ,

wobei

n=1 und 2,

m=0 und 1 und

R' einen niederen Alkylrest mit bis zu 6 C-Atomen bedeuten,

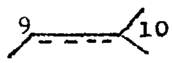

eine CC-Einfach- oder CC-Doppelbindung bedeuten und der 8-ständige Harnstoffrest α- oder β-ständig sein kann.

3. 3-(9.10-Didehydro-6-äthyl-8α-ergolinyl)-1.1-diäthylharnstoff und dessen Hydrogenmaleat
4. 3-(9.10-Didehydro-6-äthyl-8β-ergolinyl)-1.1-diäthylharnstoff und dessen hydrogenmaleat
5. 3-(9.10-Didehydro-6-n-propyl-8α-ergolinyl)-1.1-diäthylharnstoff und dessen Tartrat
6. 3-(9.10-Didehydro-6-n-propyl-8β-ergolinyl)-1.1-diäthylharnstoff und dessen Tartrat
7. 3-(9.10-Didehydro-6-isopropyl-8α-ergolinyl)-1.1-diäthylharnstoff und dessen Hydrogenmaleat
8. 3-(9.10-Didehydro-6-isopropyl-8β-ergolinyl)-1.1-diäthylharnstoff und dessen Methansulfonat
9. 3-(9.10-Didehydro-6-n-butyl-8α-ergolinyl)-1.1-diäthylharnstoff und dessen Hydrogenmaleat
10. 3-(9-10-Didehydro-6-n-butyl-8β-ergolinyl)-1.1-diäthylharnstoff und dessen Tartrat
11. 3-(6-Äthyl-8β-ergolin-l-yl)-1.1-diäthylharnstoff und dessen Phosphat
12. 3-(6-Äthyl-8α-ergolin-l-yl)-1.1-diäthylharnstoff und dessen Phosphat
13. 3-(6-n-Propyl-8β-ergolin-l-yl)-1.1-diäthylharnstoff- und dessen Tartrat
14. 3-(6-n-Propyl-8α-ergolin-l-yl)-1.1-diäthylharnstoff und dessen Phosphat
15. 3-(6-Isopropyl-8β-ergolin-l-yl)-1.1-diäthylharnstoff und dessen Hydrogenmaleat
16. 3-(6-Isopropyl-8α-ergolin-l-yl)-1.1-diäthylharnstoff und dessen phosphat
17. 3-(6-n-Butyl-8β-ergolin-l-yl)-1.1-diäthylharnstoff und dessen Phosphat
18. 3-(6-n-Butyl-8α-ergolin-l-yl)-1.1-diäthylharnstoff und dessen Phosphat
19. 3-(9.10-Didehydro-6-(2-propen-1-yl)-8α-ergolinyl)-1.1-diethylharnstoff
20. 3-(9.10-Didehydro-6-(2-propen-1-yl)-8β-ergolinyl)-1.1-diethylharnstoff
21. 3-(9.10-Didehydro-6-(2-propin-1-yl)-8α-ergolinyl)-1.1-diethylharnstoff
22. 3-(9.10-Didehydro-6-(2-propin-1-yl)-8β-ergolinyl)-1.1-diethylharnstoff
23. 3-(9.10-Didehydro-8α-(3.3-diethylureido)-6-ergolin)-propionsäureethylester
24. 3-(9.10-Didehydro-8β-(3.3-diethylureido)-6-ergolin)-propionsäureethylester
25. 3-(8α-(3.3-Diethylureido)-6-ergolin)-propionsäureethylester
26. 3-(8β-(3.3-Diethylureido)-6-ergolin)-propionsäureethylester
27. 3-(9.10-Didehydro-8α-(3.3-diethylureido)-6-ergolin)-propionitril

28. 3-(9.10-Didehydro-8β-(3.3-diethylureido)-6-ergolin)-propionitril
29. 3-(6-Cyclopropyl)-9.10-didehydro-8α-ergolinyl)-1.1-diethylharnstoff
30. 3-(6-Cyclopropyl-9.10-didehydro-8β-ergolinyl)-1.1-diethylharnstoff
31. 3-(6-Cyclopropyl-8α-ergolinyl)-1.1-diethylharnstoff
32. 3-(6-Cyclopropyl-8β-ergolinyl)-1.1-diethylharnstoff
33. 3-(6-Cyclobutyl-9.10-didehydro-8α-ergolinyl)-1.1-diethylharnstoff
34. 3-(6-Cyclobutyl-9.10-didehydro-8β-ergolinyl)-1.1-diethylharnstoff
35. 3-((6-Cyclopropyl-methyl)-9.10-didehydro-8α-ergolinyl)-1.1-diethylharnstoff
36. 3-((6-Cyclopropyl-methyl)-9.10-didehydro-8β-ergolinyl)-1.1-diethylharnstoff
37. Arzneimittel auf Basis der Verbindungen gemäß Anspruch 2 bis 36.

**Claims**

1. A process for the preparation of ergolinyl-N',N'-diethylurea derivatives of the formula

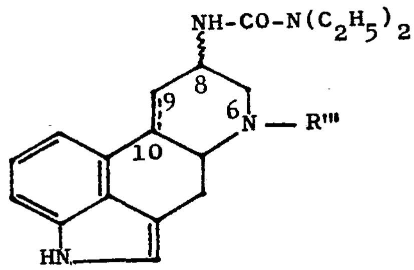

and pharmaceutically acceptable salts thereof, wherein

R''' is alkyl of 2-6 carbon atoms,

$-(CH_2)_n-CH=CH_2$, $-(CH_2)_n-C\equiv CH$, $-(CH_2)_n-COOR'$,

$-(CH_2)_n-CN$ or 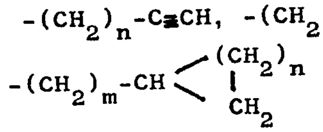 ,

n is 1, or 2,
m is 0 or 1,
R' is alkyl of 1-6 carbon atoms,

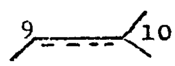

is a CC single bond or a CC double bond, and the 8-positioned urea residue can be in the α- or β-position, comprising, in a conventional manner, reacting $N^6$-alkylated lysergic acid methyl esters with hydrazine to form the corresponding hydrazide ; converting the product into the azide with nitrous acid ; forming the isocyanate by heating ; reacting the product with diethylamine ; optionally hydrogenating the 9,10-double bond ; and optionally converting the thus-obtained compounds into the salts thereof.

2. Ergolinyl-N',N'-diethylurea derivatives of the formula

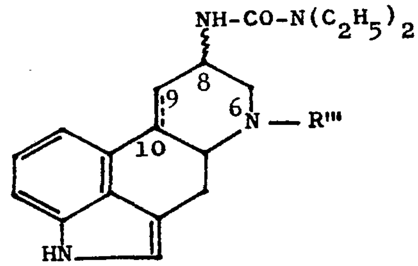

and pharmaceutically acceptable salts thereof, wherein

R''' is alkyl of 2-6 carbon atoms,

$-(CH_2)_n-CH=CH_2$, $-(CH_2)_n-C\equiv CH$, $-(CH_2)_n-COOR'$,

$-(CH_2)_n-CN$ or 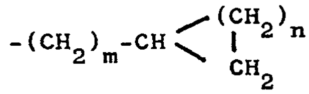

n is 1, or 2,
m is 0 or 1,
R' is alkyl of 1-6 carbon atoms,

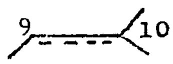

is a CC single bond or a CC double bond, and the 8-positioned urea residue can be in the α- or β-position.

3. 3-(9,10-Didehydro-6-ethyl-8α-ergolinyl)-1,1-diethylurea or the hydrogen maleate thereof.
4. 3-(9,10-Didehydro-6-ethyl-8β-ergolinyl)-1,1-diethylurea or the hydrogen maleate thereof.
5. 3-(9,10-Didehydro-6-n-propyl-8α-ergolinyl)-1,1-diethylurea or the tartrate thereof.
6. 3-(9,10-Didehydro-6-n-propyl-8β-ergolinyl)-1,1-diethylurea or the hydrogen maleate thereof.
7. 3-(9,10-Dihehydro-6-isopropyl-8α-ergolinyl)-1,1-diethylurea or the hydrogen maleate thereof.
8. 3-(9,10-Didehydro-6-isopropyl-8β-ergolinyl)-1,1-diethylurea or the methanesulfonate thereof.
9. 3-(9,10-Didehydro-6-n-butyl-8α-ergolinyl)-1,1-diethylurea or the hydrogen maleate thereof.
10. 3-(9,10-Didehydro-6-n-butyl-8β-ergolinyl)-1,1-diethylurea or the tartrate thereof.
11. 3-(6-Ethyl-8β-ergolin-I-yl)-1,1-diethylurea or the phosphate thereof.
12. 3-(6-Ethyl-8α-ergolin-I-yl)-1,1-diethylurea or the phosphate thereof.
13. 3-(6-n-Propyl-8β-ergolin-I-yl)-1,1-diethylurea or the tartrate thereof.
14. 3-(6-n-Propyl-8α-ergolin-I-yl)-1,1-diethylurea or the phosphate thereof.
15. 3-(6-Isopropyl-8β-ergolin-I-yl)-1,1-diethylurea or the hydrogen maleate thereof.
16. 3-(6-Isopropyl-8α-ergolin-I-yl)-1,1-diethylurea or the phosphate thereof.
17. 3-(6-n-Butyl-8β-ergolin-I-yl)-1,1-diethylurea or the phosphate thereof.
18. 3-(6-n-Butyl-8α-ergolin-I-yl)-1,1-diethylurea or the phosphate thereof.
19. 3-[9,10-Didehydro-6-(2-propen-1-yl)-8α-ergolinyl]-1,1-diethylurea.
20. 3-[9,10-Didehydro-6-(2-propen-1-yl)-8β-ergolinyl]-1,1-diethylurea.
21. 3-[6,10-Didehydro-6-(2-propyn-1-yl)-8α-ergolinyl]-1,1-diethylurea.
22. 3-[9,10-Didehydro-6-(2-propyn-1-yl)-8β-ergolinyl]-1,1diethylurea.
23. 3-[9,10-Didehydro-8α-(3,3-diethylureido)-6-ergolin] propionic acid ethyl ester.
24. 3-[9,10-Didehydro-8β-(3,3-diethylureido)-6-ergolin] propionic acid ethyl ester.
25. 3-[8α-(3,3-Diethylureido)-6-ergolin] propionic acid ester.
26. 3-[8β-(3,3-Diethylureido)-6-ergolin] propionic acid ethyl ester.
27. 3-[9,10-Didehydro-8α-(3,3-diethylureido)-6-ergolin] propionitrile.
28. 3-[9,10-Didehydro-8β-(3,3-diethylureido)-6-ergolin] propionitrile.
29. 3-(6-Cyclopropyl-9,10-didehydro-8α-ergolinyl)-1,1-diethylurea.
30. 3-(6-Cyclopropyl-9,10-didehydro-8β-ergolinyl)-1,1-diethylurea.
31. 3-(6-Cyclopropyl-8α-ergolinyl)-1,1-diethylurea.
32. 3-(6-Cyclopropyl-8β-ergolinyl)-1,1-diethylurea.
33. 3-(6-Cyclobutyl-9,10-didehydro-8α-ergolinyl-1,1-diethylurea.
34. 3-(6-Cyclobutyl-9,10-didehydro-8β-ergolinyl)-1,1-diethylurea.
35. 3-[(6-Cyclopropylmethyl)-9,10-didehydro-8α-ergolinyl]-1,1-diethylurea.
36. 3-[(6-Cyclopropylmethyl)-9,10-didehydro-8β-ergolinyl]-1,1-diethylurea.
37. Pharmaceutical preparations characterized in that they contain a compound according to claim 2 to 36.

**Revendications**

1. Procédé de préparation de dérivés d'(ergoline-yl)-N',N'-diéthylurée répondant à la formule générale

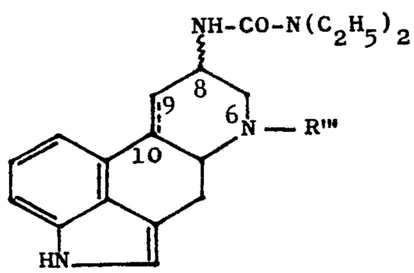

et de leurs sels, formule dans laquelle

R''' représente un radical alkyle ayant de 2 à 6 atomes de carbone, ou un groupement

$$-(CH_2)_n-CH=CH_2,\ -(CH_2)_n-C\equiv CH,\ -(CH_2)_n-COOR',$$
$$-(CH_2)_n-CN \text{ et } -(CH_2)_m-CH<\begin{matrix}(CH_2)_n\\|\\CH_2\end{matrix},$$

où

n est égal à 1 ou à 2,

m est égal à 0 ou à 1 et

R' représente un radical alkyle inférieur ayant jusqu'à 6 atomes de carbone,

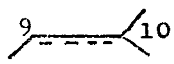

10 peut représenter une simple ou double liaison CC et le radical d'urée en position 8 peut être en position α ou β, caractérisé en ce qu'on fait réagir, d'une manière connue en soi, un ester méthylique d'acide lysergique alkyl en $N^6$ avec de l'hydrazine, ce qui donne de l'hydrazide, on convertit celui-ci en l'azide par de l'acide nitreux, on forme l'isocyanate par chauffage, puis on l'amène à réagir avec de la diéthylamine, et on hydrogène éventuellement la double liaison 9, 10 et on convertit éventuellement les composés ainsi obtenus en leurs sels.

2. Dérivés d'(ergoline-yl)-N',N'-diéthylurée répondant à la formule générale

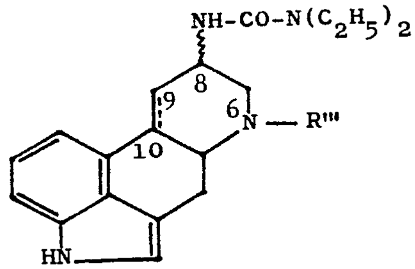

et leurs sels, formule dans laquelle

R''' peut représenter un radical alkyle ayant de 2 à 6 atomes de carbone ou un groupement

$$-(CH_2)_n-CH=CH_2,\ -(CH_2)_n-C\equiv CH,\ -(CH_2)_n-COOR',$$
$$-(CH_2)_n-CN \text{ et } -(CH_2)_m-CH<\begin{matrix}(CH_2)_n\\|\\CH_2\end{matrix},$$

où

n est égal à 1 ou à 2,

m est égal à 0 ou à 1 et

R' représente un radical alkyle inférieur ayant jusqu'à 6 atomes de carbone,

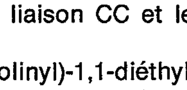

10 peut représenter une simple ou double liaison CC et le radical d'urée en position 8 peut être en position α ou β.

3. La 3-(9,10-didéshydro-6-éthyl-8α-ergolinyl)-1,1-diéthylurée et son hydrogénomaléate.
4. La 3-(9,10-didéshydro-6-éthyl-8β-ergolinyl)-1,1-diéthylurée et son hydrogénomaléate.
5. La 3-(9,10-didéshydro-6-n-propyl-8α-ergolinyl)-1,1-diéthylurée et son tartrate.
6. La 3-(9,10-didéshydro-6-n-propyl-8β-ergolinyl)-1,1-diéthylurée et son tartrate.
7. La 3-(9,10-didéshydro-6-isopropyl-8α-ergolinyl)-1,1-diéthylurée et son hydrogénomaléate.
8. La 3-(9,10-didéshydro-6-isopropyl-8β-ergolinyl)-1,1-diéthylurée et son méthanesulfonate.
9. La 3-(9,10-didéshydro-6-n-butyl-8α-ergolinyl)-1,1-diéthylurée et son hydrogénomaléate.
10. La 3-(9,10-didéshydro-6-n-butyl-8β-ergolinyl)-1,1-diéthylurée et son tartrate.
11. La 3-(6-éthyl-8β-ergoline-l-yl)-1,1-diéthylurée et son phosphate.
12. La 3-(6-éthyl-8α-ergoline-l-yl)-1,1-diéthylurée et son phosphate.

13. La 3-(6-n-propyl-8β-ergoline-I-yl)-1,1-diéthylurée et son tartrate.
14. La 3-(6-n-propyl-8α-ergoline-I-yl)-1,1-diéthylurée et son phosphate.
15. La 3-(6-isopropyl-8α-ergoline-I-yl)-1,1-diéthylurée et son hydrogénomaléate.
16. La 3-(6-isopropyl-8α-ergoline-I-yl)-1,1-diéthylurée et son phosphate.
17. La 3-(6-n-butyl-8β-ergoline-I-yl)-1,1-diéthylurée et son phosphate.
18. La 3-(6-n-butyl-8α-ergoline-I-yl)-1,1-diéthylurée et son phosphate.
19. La 3-(9,10-didéshydro-6-(2-propén-1-yl)-8α-ergolinyl)-1,1-diéthylurée.
20. La 3-(9,10-didéshydro-6-(2-propén-1-yl)-8β-ergolinyl)-1,1-diéthylurée.
21. La 3-(9,10-didéshydro-6-(2-propyn-1-yl)-8α-ergolinyl)-1,1-diéthylurée.
22. La 3-(9,10-didéshydro-6-(2-propyn-1-yl)-8β-ergolinyl)-1,1-diéthylurée.
23. L'ester éthylique d'acide 3-(9,10-didéshydro-8α-(3,3-diéthyluréido)-6-ergoline)-propionique.
24. L'ester éthylique d'acide 3-(9,10-didéshydro-8β-(3,3-diéthyluréido)-6-ergoline)-propionique.
25. L'ester éthylique d'acide 3-(8α-(3,3-diéthyluréido)-6-ergoline)-propionique.
26. L'ester éthylique d'acide 3-(8β-(3,3-diéthyluréido)-6-ergoline)-propionique.
27. Le 3-(9,10-didéshydro-8α-(3,3-diéthyluréido)-6-ergoline)-propionitrile.
28. Le 3-(9,10-didéshydro-8β-(3,3-diéthyluréido)-6-ergoline)-propionitrile.
29. La 3-(6-cyclopropyl-9,10-didéshydro-8α-ergolinyl)-1,1-diéthylurée.
30. La 3-(6-cyclopropyl-9,10-didéshydro-8β-ergolinyl)-1,1-diéthylurée.
31. La 3-(6-cyclopropyl-8α-ergolinyl)-1,1-diéthylurée.
32. La 3-(6-cyclopropyl-8β-ergolinyl)-1,1-diéthylurée.
33. La 3-(6-cyclobutyl-9,10-didéshydro-8α-ergolinyl)-1,1-diéthylurée.
34. La 3-(6-cyclobutyl-9,10-didéshydro-8β-ergolinyl)-1,1-diéthylurée.
35. La 3-((6-cyclopropyl-méthyl)-9,10-didéshydro-8α-ergolinyl)-1,1-diéthylurée.
36. La 3-((6-cyclopropyl-méthyl)-9,10-didéshydro-8β-ergolinyl)-1,1-diéthylurée.
37. Médicaments à base des composés suivant l'une des revendications 2 à 36.